# EUROPÄISCHE PATENTSCHRIFT

(11) **EP 0 697 869 B1**
(45) Veröffentlichungstag und Bekanntmachung des Hinweises auf die Patenterteilung: **18.08.1999**
(21) Anmeldenummer: 94915109.6
(22) Anmeldetag: 25.04.1994
(51) Int. Cl.: A61K 31/40, A61K 9/70

(54) **TRANSDERMALE THERAPEUTISCHE SYSTEME ZUR VERABREICHUNG VON SEROTONINAGONISTEN**
TRANSDERMAL THERAPEUTIC SYSTEMS FOR THE ADMINISTRATION OF SEROTONIN AGONISTS
SYSTEMES THERAPEUTIQUES TRANSDERMIQUES POUR L'ADMINISTRATION D'AGONISTES DE LA SEROTONINE

(30) Priorität: 06.05.1993 DE 4314976
(43) Veröffentlichungstag der Anmeldung: 28.02.1996
(73) Patentinhaber: LTS LOHMANN Therapie-Systeme GmbH, 56567 Neuwied (DE)
(72) Erfinder: LIST, Harald, D-56567 Neuwied (DE)
(74) Vertreter: Flaccus, Rolf-Dieter, Dr.
(86) Internationale Anmeldenummer: EP9401280
(87) Internationale Veröffentlichungsnummer: WO9426270

(56) Entgegenhaltungen:
- EP-A- 0 503 440
- WO-A-92/00103
- GB-A- 2 162 522
- US-A- 3 742 951
- US-A- 3 996 934
- US-A- 5 288 498

## Beschreibung

Die vorliegende Erfindung betrifft transdermale therapeutische Systeme zur systemischen Verabreichung von Wirkstoffen, ein Verfahren zur seiner Herstellung und die Verwendung dieser Systeme zur Behandlung von Krankheiten.

Eine große Zahl an Menschen leidet unter Migränekopfschmerzen, deren Pathophysiologie noch nicht vollig geklärt ist: sowohl eine starke Dilatation von Blutgefäßen des Gehirns als auch eine perivaskuläre aseptische Entzundung im Bereich von Duraarterien werden als Ursachen angesehen. Für die Regulation des Gefäßtonus wird dem Neurotransmitter Serotonin (5-Hydroxytryptamin, 5-HT) eine Schlüsselrolle zugeschrieben.

Die Migräne ist häufig begleitet von Übelkeit und Erbrechen, Licht- und Geräuschempfindlichkeit. Beim Cluster-Kopfschmerz handelt es sich um sehr intensive, intervallartig auftretende einseitige Kopfschmerzen.

Die Therapie dieser Beschwerden besteht in der Gabe von schmerzlindernden Medikamenten (Analgetika wie z.B. ASS (Acetylsalicylsäure) oder Paracetamol) und/oder gefaßtonisierenden Substanzen (Dihydroergotamin), bei Ubelkeit und Erbrechen vorzugsweise in Form von Injektionen oder rektalen Applikationen, aber auch als Dosier-Aerosol. Jedoch ist der Effekt dieser Behandlungsverfahren oft ungenügend - so ist z.B. die gefäßkonstriktorische Wirkung des Ergotamins nicht selektiv auf die Hirngefäße beschrankt und führt zu unerwünschten Nebenwirkungen.

Um die Häufigkeit von Migräneanfällen zu reduzieren, werden mit unterschiedlichem Erfolg prophylaktisch Betablocker, Calciumantagonisten, sowie 5-HT₂-Antagonisten eingesetzt.

Der zunächst naheliegend erscheinende Einsatz von Serotonin selbst zur Behandlung der Migräne ist therapeutisch nicht sinnvoll, da 5-HT auf unterschiedliche Organsysteme wirkt und zahlreiche, unerwünschte Begleiteffekte auftreten. Serotonin erhielt seinen Namen aufgrund der starken vasokonstriktorischen Wirkung. Serotoninmangel führt zu einer Gefäßdilatation, welche den Migränekopfschmerz bewirkt. Der Wirkungseintritt erfolgt über 5-HT₁-Rezeptoren im Bereich der Gefäßwände von Hirnarterien.

In den letzten Jahren wurde nun die chemische Struktur des Serotonins in vielfacher Heise modifiziert, wodurch sich Veränderungen der pharmakologischen Eigenschaften ergaben. So wurden Indolderivate synthetisiert, die eine selektive Tonisierung (Kontraktion) der Hirngefäße bewirken, verbunden mit einer schnellen Besserung der Symptome. Hierbei handelt es sich um sogenannte Serotoninagonisten mit besonderer Affinität zu 5-HT₁ ⁻Rezeptoren. Ein in diesem Zusammenhang in die Behandlung eingeführter Wirkstoff ist SUMATRIPTAN (INN) mit der Strukturformel 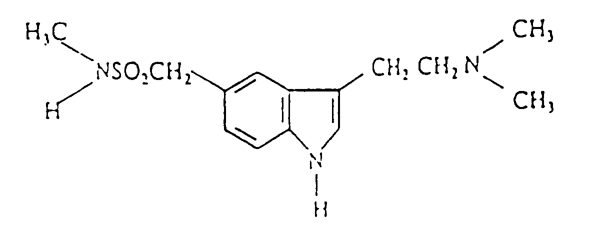 3- (2- (Dimethylamino)ethyl)- N- methyl- 1H- indol- 5-methansulfonamid

Die Synthese erfolgt gemäß dem britischen Patent 2 162 522.

Die pharmakologischen Erkenntnisse bezüglich des SUMATRIPTANS haben sich inzwischen in einer Reihe von Patenten niedergeschlagen, in denen orale, parenterale und intranasale sowie rektale Anwendungen beschrieben werden( z.B. DE 35 27 648, EP 503 440 und EP 500 086). Auf den Einsatz der Wirkstoffe in transdermalen therapeutischen Systemen wurde darin nicht Bezug genommen.

Nachteile bei der Verwendung von SUMATRIPTAN sind in der Pharmakokinetik begrundet: die Halbwertszeit von SUMATRIPTAN nach subcutaner und oraler Applikation beträgt nur ca. 2 Stunden. Die Bioverfügbarkeit bei der oralen Applikation beträgt aufgrund des prasystemischen Metabolismus lediglich 14%, während sie bei subcutaner Injektion 96% beträgt. Aufgrund der kurzen Halbwertszeit von SUMATRIPTAN kann die Migränesymptomatik bald wieder auftreten und eine erneute Applikation erforderlich machen.

Nebenwirkungen können bei der Injektion als Brennen und Rötung an der Einstichstelle auftreten, bei oraler Verabreichung ist der bittere Geschmack durch einen Überzug der Tablette zu vermeiden. Allgemein wird auch ein vorübergehendes Hitze-, Druck-, Enge- oder Schweregefühl nach der Applikation von SUMATRIPTAN beobachtet. Bei subcutaner Injektion kann auch eine Konstriktion systemischer Arterien auftreten, was bei Patienten mit Bluthochdruck oder koronarer Herzerkrankung zu beachten ist.

Der Erfindung liegt nun die Aufgabe zugrunde, eine Darreichungsform zu finden, welche die beschriebenen Nachteile vermeidet und die Effektivität des Wirkstoffes steigert.

Die oben gestellte Aufgabe wird daher gelöst durch ein transdermales therapeutisches System gemäß Anspruch 1, durch ein Verfahren zu seiner Herstellung gemäß Anspruch 10 sowie durch die erfindungsgemäße Verwendung gemäß Anspruch 11. Die Unteransprüche betreffen bevorzugte Ausführungsformen der Erfindung.

Die Erfindung bezieht sich auf ein transdermales therapeutisches System zur systemischen Verabreichung von Wirkstoffen, welches dadurch gekennzeichnet ist, daß mindestens einer der Wirkstoffe ein Serotoninagonist aus der Gruppe der Indolderivate und/oder dessen pharmazeutisch akzeptablen Salze ist. In einer besonders bevorzugten Ausführungsforn wird als Wirkstoff SUMATRIPTAN (3- (2- (Dimethylamino)ethyl)- N- methyl- 1H- indol- 5-methansulfonamid) oder eines seiner Derivate eingesetzt.

Weiterhin betrifft die Erfindung ein Verfahren zur Herstellung des transdermalen therapeutischen Systems zur systemischen Verabreichung von Wirkstoffen, wobei eine wirksame Menge mindestens eines Wirkstoffes in fester Form, in mikroverkapselter Form, in Lösung oder in Dispersion in das System eingebracht werden.

Folglich wird ein transdermales therapeutisches System eingesetzt, welches aufgrund transdermaler Absorption eine systemische Wirkung erzielt.

Ein therapeutisches System ist so definiert, daß es sich um eine arzneistoffenthaltende Vorrichtung bzw. eine Darreichungsform handelt, welche einen oder mehrere Arzneistoffe in vorausbestimmter Rate kontinuierlich über einen festgelegten Zeitraum an einen festgelegten Anwendungsort abgibt (zitiert nach Heilmann "Therapeutisches Systeme", Ferdinand- Enke- Verlag, Stuttgart 1984).

Die Vorteile eines TTS liegen in der kontinuierlichen Wirkstofffreisetzung, der verbesserten Pharmakodynamik einer Substanz mit kurzer Halbwertszeit, der gesteigerten Effizienz durch die Umgehung des First-Pass-Effektes der Leber, durch die Vermeidung der Belastung und der Risiken einer intravenösen Behandlung, Vermeidung von Nebenwirkungen im Bereich des Gastrointestinaltraktes bei oraler Medikation und der guten Akzeptanz beim Patienten. Resorptionsspitzen mit der Gefahr systemischer Nebenwirkungen werden vermieden. Im Vergleich zur sonst manchmal mehrfach erforderlichen Applikation ist eine Reduzierung der Gesamtdosis möglich.

Die bei Migräne typischerweise auftretende Übelkeit und Erbrechen lassen eine orale Applikation des Wirkstoffes nicht zu, so daß auch aus dieser Sicht die Verabreichung vermittels eines transdermalen therapeutischen Systems große Vorteile bietet. Dabei sind verschiedene Ausgestaltungen eines TTS möglich, wie sie dem heutigen Stand der Technik entsprechen.

Die technische Umsetzung von transdermalen therapeutischen Systemen ist auf der Basis folgender prinzipieller Lösungen möglich, die auch zu entsprechenden Produkten auf dem Markt geführt haben:
1. Membrangesteuerte Systeme
2. Matrixgesteuerte Systeme.

Ziel der Systeme ist es, eine kontrollierte, im allgemeinen konstante Wirkstofffreisetzung über einen definierten Zeitraum zu gewährleisten.

Für Pflaster als membran gesteuertes System sei beispielhaft auf die US-Patente 3 742 951, 3 797 494 , 3 996 934 und 3 031 894 hingewiesen. Diese Pflaster bestehen grundsätzlich aus einer Rückschicht, die eine der Oberflächen darstellt, einer für den Wirkstoff durchlässigen Haftklebeschicht, die die andere Oberfläche darstellt und letztlich einem Reservoir, das den Wirkstoff zwischen den beiden die Oberflächen bildenden Schichten enthält.

Alternativ dazu kann der Wirkstoff auch in einer Vielzahl von Mikrokapseln enthalten sein, die innerhalb einer für den Wirkstoff durchlässigen Haftklebeschicht verteilt sind. In diesem Fall kann das Kapselmaterial auch als Steuermembran wirken.

Ein Pflaster als matrixkontrolliertes System (also mit Matrix-Diffusions-Steuerung) wird z.B. in DE-PS 33 15 272 beschrieben. Es besteht aus einer undurchlässigen Rückschicht, einem damit verbundenen, besonders aufgebauten Reservoir aus einer Polymermatrix, das den Wirkstoff gegebenenfalls in einer Konzentration oberhalb der Sättigungskonzentration enthält, einer mit dem Reservoir verbundenen, für den Wirkstoff durchlässigen Haftklebeschicht, wobei eine die Haftklebeschicht abdeckende, zum Gebrauch wieder ablösbare Schutzschicht vorhanden sein kann. Ist die Reservoirmatrix selbst schon haftklebend, so kann auf die zusätzliche Haftklebeschicht verzichtet werden.

Erfindungsgemäß umfaßt das TTS als matrixkontrolliertes System in Pflasterform eine Rückschicht, ein damit verbundenes Wirkstoffreservoir mit einer die Abgabe der Wirkstoffe steuernden Polymermatrix und eine für Wirkstoffe durchlässige Haftklebeschicht zur Befestigung des Systems auf der Haut.

Auch erweist sich vornehmlich das TTS, welches in seinem Wirkstoffreservoir die Wirkstoffe in einer Konzentration oberhalb der Sättigungskonzentration enthält, als hervorragend geeignet, das der Erfindung gestellte Problem zu lösen.

In einer besonderen Ausführungsform der Erfindung kann das transdermale therapeutische System zur systemischen Verabreichung von Wirkstoffen als Wirkstoff SUMATRIPTAN (3- (2- (Dimethylamino)ethyl)- N- methyl- 1H- indol- 5-methansulfonamid) oder eines seiner Derivate enthalten.

Vorzugsweise kann ein TTS verwendet werden, welches eine von der Haftklebeschicht ablösbare Schutzschicht enthält. Als besonders geeignet zeigte sich das erfindungsgemäße TTS in Pflasterform bei Verwendung einer undurchlässigen Rückschicht.

Die Dosierung von SUMATRIPTAN bzw. einem anderen pharmakologisch akzeptablen Indolderivat muß so gewählt werden, daß während des vorgesehenen Anwendungszeitraums der effektive Serumspiegel erreicht werden. Für SUMATRIPTAN liegen diese Serumspiegel um 50 bis 70 Mikrogramm pro Liter.

Diese TTS werden verwendet zur Herstellung eines gebrauchsfertigen Arzneimittels. Dazu bedarf es der Festlegung von Parametern wie z.B. Wirkstoffwahl, Dosis, Steuerung der Freisetzung und Freisetzungsrate, Zusammensetzung des Reservoirs und Zusatz von Hilfsmitteln.

Als Hilfsmittel wird mindestens ein Vertreter der Penetrationsverbesserer, Alterungsschutzmittel, Stabilisatoren, Trägerstoffe, durchblutungsfördernder Mittel und Füllstoffe umfassenden Gruppe zugesetzt.

Als Penetrationsverbesserer kommen Carbonsäuren wie Octansäure, Stearinsäure, Ölsäure etc. in Frage. Die Verwendung der oben genannten Zusätze, welche u.a. von der Art der Wirkstoffe abhängt sind dem Fachmann bekannt.

Auch kann ein weiteres Prinzip zur Verbesserung der Permeation von Wirkstoffen durch die Haut berücksichtigt werden, nämlich die Anwendung von elektrischem Strom (Iontophorese). Die Wirkstoffe können in unterschiedlicher Form (fest, in Lösung, in Dispersion) in ein TTS eingebracht werden, sie können auch mikroverkapselt vorliegen. Der Gehalt eines derartigen Verabreichungssystems an SUMATRIPTAN liegt vorzugsweise zwischen 10 bis 200 Milligramm.

Dabei zeigt sich, daß das erfindungsgemäße TTS zur systemischen Verabreichung von Wirkstoffen sich besonders zur Herstellung eines Arzneimittels zur Behandlung von Migräne und Cluster-Kopfschmerzen eignet.

Schließlich kann es sinnvoll sein, Serotoninagonisten mit einem anderen Wirkstoff zu kombinieren - mit dem Ziel einer Wirkungspotenzierung, gegebenenfalls aber auch Reduzierung der Einzeldosen (z.B. mit Analgetika, Antimimetika, Psychopharmaka oder Sedativa).

## Patentansprüche

1. Transdermales therapeutisches System zur systemischen Verabreichung von Wirkstoffen, mit mindestens einem Serotoninagonist aus der Gruppe der Indolderivate und/oder dessen pharmazeutisch akzeptabler Salze, das in Pflasterform vorliegt und eine Rückschicht, ein damit verbundenes Wirkstoffreservoir, bei Abwesenheit enderer Steuermechanismen eine die Abgabe des Wirkstoffs steuernde Membran und eine Haftklebeeinrichtung zur Befestigung des Systems auf der Haut umfaßt. dadurch gekennzeichnet, daß das Wirkstoffreservoir die Wirkstoffe in einer Konzentration oberhalb der Sättigungskonzentration enthält, und daß es mindestens ein Hilfsmittel der Gruppe Penetrationsverbesserer, Alterungsschutzmittel, Stabilisatoren. Trägerstoffe, durchblutungsfördernde Mittel und Füllstoffe enthält.

2. Transdermales therapeutisches System zur systemischen Verabreichung von Wirkstoffen nach Anspruch 1, dadurch gekennzeichnet, daß der Wirdstoff SUMATRIPTAN (3-(2-(Dimethylamino)ethyl)-N-methyl-1H-indol-5-methansulfonamid) oder eines seiner Derivate ist.

3. Transdermales therapeutisches System nach Anspruch 1 oder 2, dadurch gekennzeichnet, daß Serotoninagonisten und/oder deren pharmazeutisch akzeptablen Salze mit weiteren Wirkstoffen kombiniert sind.

4. Transdermales therapeutisches System zur systemichen verabreichung von Wirkstoffen nach einem der Ansprüche 1 bis 3, dadurch gekennzeichnet daß mindestens einer der Wirkstoffe in mikroverkapselter Form verliegt.

5. Transdermales therapeutisches System zur systemischen Verabreichung von Wirkstoffen nach Anspruch 4, dadurch gekennreichnet, daß die Kapselwend oder des Kapselmaterial als Membran ausgebildet ist.

6. Transdermales therepeutisches System zur systemischen Verabreichung von Wirkstoffen nach einem der Ansprüche 1 bis 5, dadurch gekennzeichnet, daß es eine von der Haftklebeschicht ablösbare Schutzschicht enthält.

7. Transdermales therapeutisches System zur systemischen Verabreichung von Wirkstoffen nach einem der Ansprüche 1 bis 6, dadurch gekennzeichnet, daß die Rückschicht für die Bestandteile des Reservoirs undurchlässig ist.

8. Transdermales therapeutisches System zur systemischen Verabreichung von Wirkstoffen nach einem der Ansprüche 1 bis 7, dadurch gekennzeichnet, daß es 10 bis 200 mg SUMATRIPTAN enthält.

9. Transdermales therapeutisches System zur systemischen Verabreichung von Wirkstoffen nach Anspruch 1, gekennzeichnet durch Verwendung von elektrischem Strom als Penetrationsverbesserer.

10. Verfahren zur Hellstellung des transdermalen therapeutischen Systems zur systemischen Verabreichung von Wirkstoffen nach einem der Ansprüche 1 bis 9, dadurch gekennzeichnet, daß eine wirksame Menge mindestens eines Wirkstoffes in fester Form, in mikroverkapselter Form, in Lösung oder in Dispersion in das System eingebracht werden.

11. Verwendung des transdermalen therapeutischen Systems zur systemischen Verabreichung von Wirkstoffen nach einem der Ansprüche 1 bis 10 zur Herstellung eines Arzneimittels zur Behandlung von Migräne und Cluster-Kopfschmerzen.

## Claims

1. Transdermal therapeutic system for the systemic administration of active substances, having at least one serotonin agonist of the group comprising indole derivatives and/or its pharmaceutically acceptable salts, which system is present in the form of a patch and comprises a backing layer, an active substance reservoir connected thereto, in the absence of other controlling mechanisms a membrane which controls the release of the active substance, and a pressure-sensitive adhesive facility for fixing the system to the skin, characterized in that the active substance reservoir contains the active substances in a concentration which is above the saturation concentration, and that is contains at least one adjuvant of the group comprising penetration enhancers, anti-aging agents, stabilizers, carriers, blood flow stimulants and fillers.

2. Transdermal therapeutic system for the systemic administration of active substances according to claim 1, characterized that the active substance is SUMATRIPTAN (3-(2-(dimethyl-amino)ethyl)-N-methyl-1H-indole-5-methane sulfonamide) or one of its derivatives.

3. Transdermal therapeutic system according to claim 1 or 2, characterized in that serotonin agonists and/or their pharmaceutically acceptable salts are combined with further active substances.

4. Transdermal therapeutic system for the systemic administration of active substances according to any one of claims 1 to 3, characterized in that at least one of the active substances is present in a microencapsulated form.

5. Transdermal therapeutic system for the systemic administration of active substances according to claim 4, characterized in that the wall or the material of the capsule is formed as a membrane.

6. Transdermal therapeutic system for the systemic administration of active substances according to any one of claims 1 to 5, characterized in that it has a protective layer which can be removed from the pressure-sensitive adhesive layer.

7. Transdermal therapeutic system for the systemic administration of active substances according to any one of claims 1 to 6, characterized in that the backing layer is impermeable to the components of the reservoir.

8. Transdermal therapeutic system for the systemic administration of active substances according to any one of claims 1 to 7, characterized in that it comprises 10 to 200 mg of SUMATRIPTAN.

9. Transdermal therapeutic system for the systemic administration of active substances according to claim 1, characterized by the use of electrical current as penetration enhancer.

10. A process for the production of the transdermal therapeutic system for the systemic administration of active substances according to any one of claims 1 to 9, characterized in that an effective amount of at least one active substance is introduced into the system in solid form, in microencapsulated form, in solution or in dispersion.

11. The use of the transdermal therapeutic system for the systemic administration of active substances according to any one of claims 1 to 10 for producing a pharmaceutical product for the treatment of migraine and cluster headache.

## Revendications

1. Système thérapeutique transdermique pour l'administration systémique de principes actifs, avec au moins un agoniste de la sérotonine du groupe des dérivés indoliques et/ou leurs sels pharmaceutiquement acceptables, qui se trouve sous forme d'emplâtre et qui comporte une couche dorsale, un réservoir de principes actifs relié à celle-ci, en cas d'absence d'autres mécanismes de contrôle, une membrane contrôlant la libération du principe actif et un dispositif d'adhérence pour la fixation du système sur la peau, caractérisé en ce que le réservoir de principes actifs contient les principes actifs en une concentration supérieure à la concentration de saturation et en ce qu'il contient au moins un adjuvant du groupe des agents améliorant la pénétration, des agents de protection contre le vieillissement, des agents stabilisateurs, des supports, des agents favorisant la circulation sanguine et des matières de charge.

2. Système thérapeutique transdermique pour l'administration systémique de principes actifs selon la revendication 1, caractérisé en ce que le principe actif est le SUMATRIPTAN (3-(2-(diméthylamino)éthyl)-N-méthyl-1H-indole-5-méthanesulfonamide) ou un de ses dérivés.

3. Système thérapeutique transdermique selon la revendication 1 ou 2, caractérisé en ce que les agonistes de la sérotonine et/ou leurs sels pharmaceutiquement acceptables sont combinés à d'autres principes actifs.

4. Système thérapeutique transdermique pour l'administration systémique de principes actifs selon l'une des revendications 1 à 3, caractérisé en ce qu'au moins un des principes actifs se trouve sous forme microencapsulée.

5. Système thérapeutique transdermique pour l'administration systémique de principes actifs selon la revendication 4, caractérisé en ce que la paroi de la capsule ou le matériau de la capsule se présente sous forme d'une membrane.

6. Système thérapeutique transdermique pour l'administration systémique de principes actifs selon l'une des revendications 1 à 5, caractérisé en ce qu'il contient une couche de protection détachable de la couche adhésive.

7. Système thérapeutique transdermique pour l'administration systémique de principes actifs selon l'une des revendications 1 à 6, caractérisé en ce que la couche dorsale est imperméable aux constituants du réservoir.

8. Système thérapeutique transdermique pour l'administration systémique de principes actifs selon l'une des revendications 1 à 7, caractérisé en ce qu'il contient 10 à 200 mg de SUMATRIPTAN.

9. Système thérapeutique transdermique pour l'administration systémique de principes actifs selon la revendication 1, caractérisé par l'utilisation de courant électrique à titre d'agent améliorant la pénétration.

10. Procédé de préparation d'un système thérapeutique transdermique pour l'administration systémique de principes actifs selon l'une des revendications 1 à 9, caractérisé en ce qu'on introduit une quantité efficace d'au moins un principe actif sous forme solide, sous forme micro-encapsulée, en solution ou en dispersion dans le système.

11. Utilisation du système thérapeutique transdermique pour l'administration systémique de principes actifs selon l'une des revendications 1 à 10 pour la préparation d'un médicament pour le traitement de la migraine et de la céphalée histaminique (cluster headache).
